# NEW EUROPEAN PATENT SPECIFICATION

(11) **EP 2 381 923 B2**
(45) Date of publication and mention of the opposition decision: **07.06.2023**
(45) Mention of the grant of the patent: 01.08.2018
(21) Application number: 09796558.6
(22) Date of filing: 21.12.2009
(51) Int. Cl.: A61K 9/00, A61K 31/4025, A61K 31/11, A61M 11/02, A61M 15/00, A61M 15/08

(54) **INTRANASAL DESMOPRESSIN ADMINISTRATION**
SICHERE VERABREICHUNG VON DESMOPRESSIN
ADMINISTRATION INTRANASALE DE DESMOPRESSINE

(30) Priority: 22.12.2008 US 139871 P
(43) Date of publication of application: 02.11.2011
(60) Divisional application: 18174439.2
(73) Proprietor: Acerus Pharmaceuticals USA, LLC, Fort Washington, PA 19034 (US)
(72) Inventor: FEIN, Seymour, New Canaan CT 06840 (US)
(74) Representative: Simmons & Simmons
(86) International application number: PCT/US2009/068962
(87) International publication number: WO 2010/075266

(56) References cited:
- WO-A1-95/01183
- US-A1- 2005 158 247
- LAW S L ET AL: "Preparation of desmopressin-containing liposomes for intranasal delivery", JOURNAL OF CONTROLLED RELEASE 20010223 NL LNKD- DOI:10.1016/S0168-3659(00)00369-2, vol. 70, no. 3, 23 February 2001 (2001-02-23), pages 375-382, XP002608762, ISSN: 0168-3659
- FABRIZIO B ET AL: "In vitro permeation of desmopressin across rabbit nasal mucosa from liquid nasal sprays: The enhancing effect of potassium sorbate", EUROPEAN JOURNAL OF PHARMACEUTICAL SCIENCES, ELSEVIER, AMSTERDAM, NL, vol. 37, no. 1, 11 April 2009 (2009-04-11) , pages 36-42, XP025992154, ISSN: 0928-0987, DOI: DOI:10.1016/J.EJPS.2008.12.015 [retrieved on 2008-12-31]

## Description

### FIELD OF THE INVENTION

The disclosed invention relates to compositions and devices for intranasal administration of desmopressin so as to induce antidiuretic effects such as voiding postponement in a patient while minimizing the likelihood that the patient suffers from hyponatremia.

### BACKGROUND OF THE INVENTION

Desmopressin (1-desamino-8-D-arginine vasopressin, dDAVP^{®}) is an analogue of vasopressin. Desmopressin has decreased vasopressor activity and increased anti-diuretic activity compared to vasopressin, and, unlike vasopressin, does not adversely effect blood pressure regulation. This enables desmopressin to be used clinically for anti-diuresis without causing significant increases in blood pressure. Desmopressin is commercially available as the acetate salt and is commonly prescribed for primary nocturnal enuresis (PNE) and central diabetes insipidus.

Desmopressin is a small peptide and is characterized by poor bioavailability. For treatment of severe illness such as cranial diabetes insipidus, it may be administered intravenously or subcutaneously, routes which essentially are 100% bioavailable. When taken in the commercialized dose forms of oral, sublingual and nasal spray delivery, bioavailability is poor. Oral doses (pills) have a bioavailability far less than one percent, produce a wide range of blood concentrations of the drug depending on many factors, and produce a generally indeterminate duration of antidiuretic effect. Administration of desmopressin via the buccal mucosa and trans dermally also have been suggested. Intranasal dosage forms have been approved for treatment of PNE, but the commercially available product (Minirin^{™}) has now been declared to be unsafe for this use.

Hyponatremia is a condition in which the sodium concentration in the plasma is too low, e.g. below about 135 mmol/L. Severe hyponatremia can result in electrolyte abnormalities that can cause cardiac arrhythmias, heart attack, seizures or stroke. A hyponatremic state in patients administered desmopressin therapy occurs when the water channels in the kidneys of the patient are activated by the drug and the patient consumes aqueous liquids. This can but does not always result in lowering of blood osmolarity, lowering of sodium concentration, and consequent neurological damage. Some patients on a desmopressin regimen exhibit hyponatremia suddenly after having taken the drug without incident for long periods. Others develop the condition very early in the therapeutic regime. In short, the incidence of hyponatremia has largely been regarded as a stochastic side effect of the antidiuretic desmopressin therapy, avoidable only by avoidance of fluid intake while under the drug's effect.

Recent deaths from hyponatremia have been attributed to over intake of water while under the influence of desmopressin. As a result of these experiences, the U.S. Food & Drug Administration recently has warned physicians that use of desmopressin should be curtailed, that it is no longer indicated as appropriate for certain conditions, such as primary nocturnal enuresis (PNE), and has "Black Boxed" the drug. The recent warning stated that "[c]ertain patients, including children treated with the intranasal formulation of [desmopressin acetate] for primary nocturnal enuresis (PNE), are at risk for developing severe hyponatremia that can result in seizures or death."

Currently, approved labeling for desmopressin administered intranasally for treatment of PNE indicates bioavailability in the formulation is 3-5% and recommends dosing 10-40 micrograms per day. The average maximum plasma/serum concentrations achieved (Cₘₐₓ) with a typical intranasal dose (20 µg, 10 µg in each nostril) of desmopressin for PNE is at least approximately 20-30 pg/ml, based on 3-5% bioavailability with a 6 to 10 fold range. While existing formulations of desmopressin have proven to be adequate for many patients when used for these clinical indications, variable efficacy and occasional hyponatremic episodes continue to be problems related to the aforementioned variability.

U.S. Patent 7,405,203 discloses antidiuretic therapy methods and desmopressin dosage forms. WO05/01183 discloses a nasal spray comprising a reservoir of desmopressin and a permeation enhancer (such as bile salts). It discloses that the threshold plasma concentration for activation of the antidiuretic effect of desmopressin in humans is very low, less than about 1.0 pg/ml, and based in part on this observation, proposes the use and teaches how to make and use novel low dose desmopressin dosage forms that can substantially avoid the stochastic and unpredictable onset of hyponatremia. This is accomplished by administration of a very low dose of the drug, a dose sufficient to raise the desmopressin concentration in the blood only slightly above its threshold (e.g., about 0.5 pg/ml) from about 1.0, to about 10, and perhaps as high as 15 pg drug per ml of blood in some patients, but preferably no greater than about 10 pg/ml. This low concentration was discovered to be sufficient to induce potent antidiuretic effects of limited and controlled duration. Thus, the low blood concentration in combination with the known, approximate 90+ minute half life of desmopressin in a healthy person can function to control the "off switch" of the drug's activity and thereby to limit the duration of antidiuresis. This very significantly reduces the likelihood that the patient will drink sufficient liquids during the interval the drug is physiologically active such that the patient's homeostasis mechanisms are overwhelmed and blood sodium concentration falls to dangerous levels.

For example, in the treatment of nocturia (awakening from sleep to void at night) a low dose producing, e.g., a blood concentration of 5-7 pg/ml, can be administered at bed time. In less than about one half hour, desmopressin concentration is at its maximum of about 7 pg/ml, and urine production is suppressed. After two hours (one half life) the desmopressin concentration falls to about 3.5 pg/ml, at 3.5 hr (second half life), concentration is about 1.75, at 5 hr, approximately 0.85, and at 6 hours the concentration has fallen below the activation threshold (in many patients about 0.5 pg/ml) and the patient is making urine normally. If he retires at 11:00 PM, during the first six hours the patient makes little or no urine, his bladder is essentially empty, and his urge to urinate is accordingly suppressed. By 5 AM or so, urine production is restored and in an hour or two the patient wakes to urinate. As another example, a small dose, say 2-3 pg/ml administered intranasally or through a trans or intradermal patch, can induce safe antidiuresis for about three hours before normal urine production is restored.

Intranasal administration is an attractive dosage route, and if one could formulate an intranasal dosage form that would consistently produce a desmopressin blood concentration within or near the desired low dose range disclosed in the '203 patent, the incidence of the hyponatremia side effect would be reduced or eliminated, and the drug could be used safely as a convenience, as well as for the management of serious and bothersome conditions. While it clearly is within the skill of the art to produce a low dose intranasal desmopressin formulation that will be serviceable and induce safe antidiuresis reproducibly, the ideal intranasal dose form would, from one administration to the next, and from batch to batch, consistently produce a blood concentration within a relatively narrow target blood concentration range. It also would be desirable to formulate such a product so as to minimize the chances of abuse (multiple dosing) that could lead to antidiuresis of longer duration and potentially the development of hyponatremia.

### SUMMARY OF THE INVENTION

The disclosed invention provides a convenient, intranasal desmopressin safety dispenser for inducing in members of a target patient population an antidiuretic effect while reducing the risk that a member of the population may develop hyponatremia. The dispenser comprises a reservoir having disposed therein a composition comprising a preparation of desmopressin and a nasal membrane permeation enhancer namely cyclopentadecanolide in an amount sufficient to constitute multiple drug doses. The reservoir is in communication with an outlet and is fitted with a pump, preferably a disposable pump, and preferably one that can be actuated manually, such as a squeeze bottle actuated dispenser, or a plunger pump fitted onto a glass bottle. The pump enables serially dispensing multiple metered doses from the reservoir through the outlet in the form of a spray into a nostril or nostrils of a patient so as to deposit a dose of consistent size onto an intranasal mucosal or other surface.

Each spray comprises a multiplicity of droplets, preferably with an average volume distribution in the range of 20 µm for D10 to about 300 µm for D90. This means that about 10% of the droplets are smaller than about 20 µm in diameter and 90% are smaller than 300 µm in diameter. Each spray dose is preferably of a weight and desmopressin concentration such that it comprises between 0.5 ng desmopressin per kilogram of the patient's body weight and 75 ng desmopressin per kilogram of the patient's body weight. The spray is characterized by a desmopressin bioavailability greater than about 5%, that is, between about 5% and 25% of the active in the composition actually enters the patient's bloodstream and contributes to the drug effect, and the remainder is degraded, typically by digestion. Generally, the higher the bioavailability of a spray, the less desmopressin per spray needs to be delivered into a nasal cavity, and vice versa, the goal being to achieve more consistently a target desmopressin maximum blood concentration (Cₘₐₓ) in members of the patient population.

In accordance with the claimed invention, the combination of properties of the spray dispenser and the composition it contains enables respective doses of spray to be effective to restrict the concentration of desmopressin produced in the bloodstream of patients, on a per kilogram basis, to a relatively narrow range, thereby to achieve a relatively consistent, time limited duration of antidiuresis. Stated differently, respective successive spray doses establish in a patient by drug transport across intranasal mucosal membranes a Cₘₐₓ of desmopressin which is relatively consistent. The amount of drug delivered to the bloodstream for repeated doses from the same dispenser to the same person preferably should differ no more than 100%, and preferably less than 50%. The dispenser's coefficient of variation is similar to the coefficient of variation of Cₘₐₓ produced by serial *subcutaneous* doses of desmopressin designed to achieve the same target Cₘₐₓ. Preferably, respective successive spray doses are sufficient to establish in a patient by intranasal delivery a Cₘₐₓ of desmopressin having a coefficient of variation within about 50%, more preferably about 25%, of the coefficient of variation of Cₘₐₓ produced by a subcutaneous dose of desmopressin designed to achieve the same target Cmax.

This consistency of bioavailability also is reflected in another property of dispensers of the invention, namely, they serve to establish in a patient by drug transport across intranasal mucosal membranes delivery of blood concentrations of desmopressin substantially directly proportional to the mass of desmopressin dispensed into the nostril(s) of a said patient. This permits self titration of the length of antidiuresis desired by a patient. Generally, the desmopressin Cₘₐₓ is directly proportional to the amount of nasally administered desmopressin over a Cmax ranging from 0.5 pg/ml to 10.0 pg/ml.

The value of the target Cₘₐₓ may be varied, depending on the duration of the antidiuretic interval the dispensed composition is designed to induce. For example, a product designed for a 7-8 hour interval of urine production suppression might be designed to deliver a Cₘₐₓ of no more than 15 +/- 3 pg/ml. Thus, by way of illustration, a 7 hour product designed for children might have a bioavailability of 20% and a desmopressin load per spray of 0.75 µg or 750 ng. This would mean that about 150 ng of drug would reach the patient's bloodstream, and that a 33 kg (-75 lb.) child would achieve the target Cₘₐₓ of about 15 pg/ml. Another embodiment of the same product might have a bioavailability of 10% and a desmopressin load per spray of 1.5 µg or 1500 ng, again producing about 150 ng drug in the patient's bloodstream and the target Cₘₐₓ of about 15 pg/ml. Another exemplary product may be designed for a 3-4 hour urine interruption and might deliver a Cₘₐₓ of no more than about 3 pg/ml. Such a product, designed, for example, for use by women averaging 60 kg (-130 lb.), might be 25% bioavailable and comprise a 250 ng desmopressin load per spray, or 15% bioavailable with a 350 ng load. In both cases, the bioavailable dose would be about 50 ng desmopressin, and the Cₘₐₓ about 3 pg/ml.

Alternatively, a single dispenser which delivers, e.g., 200 ng or 500 ng per spray, when used in accordance with package insert or physician instructions, may serve to achieve, for example, different durations of antidiuresis in the same person or the same duration of antidiuresis in a 75 kg child or 150 kg adult, simply by varying the number of spays delivered per administration event. Typically, about 20 minutes after administration of the pharmaceutical composition of the present invention, the mean urine output per minute in a treated individual decreases to less than about 4 ml/minute, preferably less than about 1 ml/min, and stays in this low range for a desired time period, such as 180 minutes, 240 minutes, 300 minutes, 360 minutes, or 420 minutes. About twenty minutes after administration, the mean urine osmolarity is greater than about 300 mOsmol/kg and remains at high concentration for a period of time ranging up to 180 minutes, 240 minutes, 300 minutes, 360 minutes, or 420 minutes.

A primary and important property of the dosage forms of the claimed invention is that they consistently deliver per spray a maximum blood concentration within a relatively narrow time and dose range, and therefore avoid or minimize accidental delivery of a larger dose resulting in a longer than expected antidiuretic effect and the possibility of induction of hyponatremia. Consistent delivery, as the phrase is used herein, should be taken to mean repeatable within a range similar to the range observed when administering very low doses of desmopressin by subcutaneous injection, or perhaps somewhat greater. Such consistency generally is achieved more easily exploiting formulations with higher bioavailability, and accordingly a bioavailability of at least 5%, preferably at least 10%, more preferably at least 15%, and preferably even higher is preferred. Higher bioavailability is achieved by exploiting formulation technology, especially the use of permeation enhancers, and by chemical engineering of the spray composition as disclosed herein.

In one embodiment, the dispenser may further comprise means for blocking dispensing of a second desmopressin spray, or series of sprays above a certain dose, e.g., above about a dose sufficient to produce a blood concentration above about 10 to 12 pg/ml, for a predetermined time interval after dispensing a first dose. This can be achieved passively as a consequence of the design of the spray mechanism as disclosed, for example, in U.S. patent number 7,335,186. Alternatively, an active timer, powered by a battery, mechanical spring, or compressed gas within the dispenser, may be included together with mechanisms known per se designed to preclude a second dispensing until passage of a predetermined interval, e.g., 8 hours, or somewhere between 6 to 24 hours. Such a mechanism can discourage abuse of the product and further minimize the chances that a patient may inadvertently or intentionally self-induce antidiuresis for too long.

In various embodiments, the dispenser may be formulated to induce antidiuresis in a target patient population for less than six hours, for between 2 and 4 hours, or for between 4 and 7 hours. Maintaining the antidiuretic state for more than about 8 hours is not recommended. The target patient population may be, for example, children, children weighing less than 35 kg, children weighing between 35 and 50 kg, adult females, females weighing between 50 and 75 kg, adult males, males weighing between 70 and 85 kg, or males weighing more than 85 kg.

The permeation enhancer for use in the claimed invention is cyclopentadecanolide, known in the trade as CPE-215.

The claimed invention provides uses of the safety dispenser to induce an antidiuretic effect. The dispenser and desmopressin formulation may comprise any of the properties described herein. For example, the desmopressin Cₘₐₓ is directly proportional to the amount of nasally administered desmopressin over a Cₘₐₓ ranging from 0.5 pg/ml to 10.0 pg/ml. The value of the target Cₘₐₓ may be varied, depending on the duration of the antidiuretic interval the dispensed composition is designed to induce. For example, use of a safety dispenser described herein may produce antidiuresis for less than about 8 hours, less than about 6 hours, for between about 2 and 4 hours, or for between about 4 and 7 hours. Another exemplary use of a product may be designed to deliver a Cₘₐₓ in a patient of no more than 15 pg/ml, 10 pg/ml, 7 pg/ml, or 3 pg/ml.

### BRIEF DESCRIPTION OF THE DRAWINGS

Figure 1 is a graph of mean urine output vs. time (600 minutes) for men and women treated with 2000 ng intranasally administered desmopressin composition of the invention.
Figure 2 is a graph of mean urine osmolarity vs. time for men and women treated with the same composition of the invention.

### DESCRIPTION

The term bioavailability is used to describe the fraction of an administered dose of drug that reaches the systemic circulation. By definition, when a medication is administered intravenously, its bioavailability is 100%. However, when administered via other routes, such as intranasally, bioavailability decreases due to incomplete absorption and other factors. Thus, bioavailability is a measurement of the extent of a therapeutically active drug that reaches the systemic circulation and is available at the site of action. It differs widely depending on chemical and physical properties of the drug in question and its route of administration. A quantity of the composition of the invention administered intranasally refers to the quantity that exits the spray nozzle and enters the nostril(s). A quantity of the composition of the invention delivered refers to the quantity that actually reaches the bloodstream, i.e., becomes bioavailable. Proteins and peptides are relatively large and fragile molecules whose activity generally depends on their tertiary structure. The bioavailability of protein and peptide therapeutics administered other than parenterally is notoriously poor and variable.

The coefficient of variation, Cᵥ, as used herein, refers to a number expressed as a percentage that is a measure of the variability of the amount of and rapidity with which active drug gets into the bloodstream when the same drug dose form is administered the same way, to the same person over many administrations or to many different persons. A coefficient of variation can be measured for Cₘₐₓ, Tₘₐₓ, or AUC. It is often expressed as the ratio of the standard deviation of a set of measurements to the mean of those measurements. Generally, intravenous or subcutaneous administration of any drug will have an inherently smaller Cᵥ as compared with transdermal or oral administration. Intranasal administration of desmopressin is characterized not only by poor bioavailability, but also by a high Cᵥ. Thus, the commercially available Minirin^{®} nasal spray product on the basis of Cₘₐₓ achieved per nasal spray dose has a high Cᵥ, 2 to 2.5 times that of subcutaneous injection. Thus, two patient's of the same weight using the same drug ostensibly the same way may experience widely varying blood concentrations of desmopressin, as measured, for example, using Cₘₐₓ, which may have a range of six to ten fold.

The coefficient of variation is calculated from measured blood concentrations. Accordingly, the imprecision of the analytical technique used to make the measurements comprising the raw data will contribute to Cᵥ. An assay with a large inherent error bar will produce a higher measured Cᵥ than an assay with a smaller error bar. When the measurements are made at the lower end of the dynamic range of an assay, where the standard deviation of the measurements is larger, Cᵥ as calculated based on the data will be larger than the Cᵥ of a larger dose of the same drug administered the same way and measured using the same assay.

The term "permeation enhancer," as used herein, refers to one or a mixture of substances which when formulated together with a peptide active, such as desmopressin, have the effect of increasing the fraction of the peptide applied to a nasal mucosal surface that traverses the mucosal membrane and enters the bloodstream, i.e., increases bioavailability. Many such permeation enhancers are known, as described herein. Generally, the addition of a permeation enhancer to a peptide drug formulation designed for intranasal administration will increase the fraction of peptide that reaches the circulation by at least about 25%, preferably at least 50%, and most preferably at least about 100%. Thus, consider two intranasal formulations of identical composition except composition 1 has no enhancer and composition 2 comprises an additional substance. If composition 1, when administered, results in a blood concentration of 50 pg/ml, the substance falls within the definition of an enhancer if composition 2 results in a blood concentration of at least 62.5 pg/ml (25% improvement). A preferred permeation enhancer would produce a blood concentration of about 100 pg/ml (100% improvement).

The invention disclosed herein provides improvements in desmopressin nasal spray devices characterized by delivering through the nasal mucosal surfaces and into the circulation of a *more consistent* as well as a *lower* desmopressin dose so as to induce a predetermined time-limited antidiuretic effect. The nasal spray drug product contains desmopressin and a mucosal permeation enhancer which functions to promote passage of the peptide drug through the nasal mucosa. The active typically is dissolved or suspended in solutions or mixtures of excipients (e.g., preservatives, viscosity modifiers, emulsifiers, buffering agents, etc.) in a pressurized, but preferably non-pressurized, dispenser that delivers a specifically controlled amount of spray containing a metered dose into one or both nostrils. The dose typically is metered by the spray pump, which is typically finger or hand actuated. The nasal spray is designed for discharge of multiple spray doses, e.g., 10 to 100 or more. It may be designed to administer the intended dose with multiple sprays, e.g., two sprays, e.g., one in each nostril, or as a single spray, or to vary the dose in accordance with the weight, sex, or maturity of the patient, or to permit variation by the patient of the duration of antidiuresis.

The object of the design of the safety spray device is to assure to the extent possible that a *consistent* low concentration of desmopressin (the "target concentration") is delivered to the bloodstream, e.g., generally not more that an amount sufficient to produce a maximum blood concentration of 15 +/-3 pg/ml, and preferably less than 10 pg/ml. In many cases the device will deliver an amount of drug which achieves a blood concentration of 5 +/-3 pg/ml or less.

The technical difficulty of achieving this goal is presented by the low and variable bioavailability of intranasally administered peptides, including desmopressin, by the very small amounts of active being administered, and by the low target blood concentrations. To promote consistent bioavailability, the concentration of active drug ingredient per spray and the mass (amount or load) of active per spray must be controlled to control precisely the amount of active that enters a nasal passage. This involves formulation of the drug and selection of design parameters of the pump spray using known methods. However, the amount of active that reaches the nasal mucosa can depend, upon other factors, on the physical composition of the spray, i.e., total amount injected, fluid properties such as viscosity, the momentum of the spray, and its droplet size distribution. These properties also are controlled by the chemistry of the formulation and spray nozzle characteristics. Overlaid on these factors determining bioavailability is that only a portion of the fraction of active reaching the mucosa successfully traverses this membrane and enters the bloodstream. Unabsorbed drug is swallowed or otherwise degraded and is not bioavailable. Trans-mucosal passage of peptides is enhanced by including in the formulation certain substances that act as permeation enhancers. Of course, inconsistent spray procedure and the patient's particular nasal anatomy also play a part, but the inconsistency in drug uptake due to these factors cannot be controlled except by physician and/or packaging instructions for use that are explicit and clear and followed by the patient.

Applicants discovered that it is possible to safely administer desmopressin by producing an intranasal spray dispenser exploiting these design principles in combination as claimed herein.

A product designed, for example, to treat nocturia (urinary voiding at night interrupting sleep) in adults, to treat bed wetting in children (primary nocturnal enuresis), or to prevent bed wetting by a person suffering from incontinence, ideally would be taken by the patient after urinating at bedtime. Ideally the dose would suppress urine production for at least five hours, ideally six to six and a half, and possibly as much as eight. A product designed to interrupt urine production for a few hours during the day, such as to take a car trip for three or four hours, should interrupt urine production for two-three hours. At the end of the antidiuretic interval the healthy body seeks homeostasis rapidly and urine is produced normally. Thus, the urge to urinate returns in the next hour or next few hours. The products described herein of course also may be used, preferably under the care of a physician, for more serious disease such as central diabetes insipidus.

Of course, all of the times recited above are approximate, as the duration of antidiuresis achieved in a given person taking a given dose will have a certain inevitable variability. However, the intent and effect of the practice of the invention is to assure to the extent possible that a dose designed to last overnight does not in fact produce only three hours of antidiuresis, resulting in early waking, or involuntary voiding. More important, the effect of practice of the invention is to minimize the possibility that the interval of antidiuresis lasts unexpectedly long, e.g., 10 or 12 hours, resulting in an awake patient drinking liquids, and possibly developing hyponatremia.

The urine production suppression begins when the patient's desmopressin blood concentration exceeds the activation threshold of the water channels in the proximal kidney tubules, and ends when the concentration falls below that threshold. The exact concentration which is sufficient in a given individual to activate the water channels will vary, and it is so low that it is hard to measure with precision, but as disclosed in U.S. Patent 7,405,203, experiments suggest the threshold is somewhat less than 1.0 pg/ml, or about 0.5 pg/ml, and possibly somewhat lower.

Table 1 illustrates certain important features of various embodiments of the invention. Referring to the Table, it discloses dosage parameters, ranges of maximum expected blood concentrations, the average weight of members of various patient populations, and expected durations of antidiuresis for each population. All listed dose forms are exemplary only and should not be regarded as limiting, except as otherwise indicated in the claims. All these products assume that one spray equals one dose. Of course multiple sprays could be employed to achieve the same dose and this may be desirable as promoting consistent uptake.

The first two products exemplify alternative ways to achieve antidiuresis for the treatment of nocturia in adult males. Both generate a Cₘₐₓ of about 5-8 pg/ml, but the first has a 10 % bioavailability and delivers 1.0 to 1.6 µg desmopressin per spray, while the second has a bioavailability of about 20%, so requires only about half as much active per spray. Both deliver about 100 to 160 ng of drug to the patient's bloodstream, and this amount circulates to produce the desired blood concentration (Cₘₐₓ). Exemplary product 3 is designed to treat enuresis in children. If the child has an average weight of 35 kg, he or she will experience 5 to 7 hours of antidiuresis with an intranasal dose of 300-400 ng and a 15% bioavailability. This will deliver 45-70 ng desmopressin to the child's circulation and produce the desired 5-8 pg/ml concentration that will fall below the threshold concentration as normal clearance mechanisms reduce drug concentration until the threshold is passed five to seven hours later. Exemplary product 4 is designed to induce short duration urine suppression in, e.g., females averaging 60 kg. In this case, the interval desirably is short, e.g., about three hours. This can be achieved by intranasal administration of a dose that will produce a Cₘₐₓ of 1-2 pg/ml. This blood concentration can be achieved reliably with proper use of a dispenser delivering a 100-200ng load characterized by a 15% bioavailability. Products 5 and 6 illustrate still other products designed for treatment of nocturia or other therapies involving temporary suppression of urine production in a 60 kg woman or a 200 kg man.

**Table 1**

| | Patient Population | Duration of Antidiuresis | Mass of Drug per Spray | Bioavailability | Drug Delivered to Bloodstream | Cmax |
|---|---|---|---|---|---|---|
| 1 | 70 kg adults | 5-7 hr | 1.0-1.6 µg- | 10% | 100-160 ng | 5-8 pg/ml |
| 2 | 70 kg adults | 5-7 hr | 500-800 ng | 20% | 100-160 ng | 5-8 pg/ml |
| 3 | 35 kg children | 5-7 hr | 300-480 ng | 15% | 45-70 ng | 5-8 pg/ml |
| 4 | 60 kg adult females | 3 hr | 100-200 ng | 15% | 15-35 ng | 1-2 pg/ml |
| 5 | 60 kg adult females | 5-7 hr | 400-700 ng | 20% | 80-140 ng | 5-8 pg/ml |
| 6 | 100 kg adult males | 5-7 hr | 3-4.5 µg | 5% | 140-220 ng | 5-8 pg/ml |

Turning now to the details of the design of the safety dispenser, suitable drug reservoir's such as glass bottles and plastic squeeze bottles are widely available and used for pharmaceutical dispensing. Preferably the reservoir and the spray pump are disposable. Finger actuated pump sprays comprising plastic parts and metal springs are available commercially, for example, from Pfeiffer of America, Inc, Princeton New Jersey. These are available in designs to control drop size distribution to meet various specifications. For use in intranasal products the pumps typically deliver a 100 µl load in a narrow spray pattern, although in various embodiments of the invention the volume per spray may be varied, e.g., between 50 µl and 150 µl. Many different such metered drug pump designs can be adapted for use in the invention. Non limiting examples are disclosed in U.S. patent numbers 4,860,738, 4,944,429, 6,321,942, 6,446,839, 6,705,493, 6,708,846, 6,772,915, and 7,182,226.

The currently preferred spray apparatus is sold as the Pfeiffer APF pump and is fitted to a 5.0 ml glass bottle. It delivers a metered, 100 µl load in a narrow spray pattern. Preferably, to promote consistency, the spray delivers the active formulation as a multiplicity of droplets with an average volume distribution in the range of 20 µm for D10 to about 300 µm for D90. This means that about 10% of the droplets are smaller than about 20 µm in diameter and 90% are smaller than 300 µm in diameter. Other distributions may be used. Very small droplets tend to be inhaled and may or may not reach the circulation. Large droplets may not penetrate the nostril lumen sufficiently and may result in leakage and loss. Such metered pumps assure that, with proper injection protocol, each use results in expelling a metered amount and that a relatively constant amount ends up in contact with the nasal mucosal surface.

The claimed composition disposed within the reservoir comprises desmopressin, also called Anti-Diuretic Hormone, 1-desamino-8-D-arginine vasopressin, or dDAVP. It is a water soluble vasopressin analog having a molecular weight of 1069.23. Drug grade material is widely commercially available as the acetate salt. The term desmopressin, as used herein, refers to 1-desamino-8-D-arginine vasopressin and all other such analogs having antidiuretic activity, including analogs of active allelic variants of human vasopressin, and including other anions. See, for example U.S. 3,980,631, and U.S. 4,148,787.

The claimed composition also necessarily includes at least one substance that acts as a permeation enhancer, that is, a substance which increases the net peptide transport across the mucosal membranes from the nasal lumen to the capillary bed behind it. Many permeation enhancers are known in the art, and there are many ways to formulate such enhancers with peptide drugs so as to effectively increase their bioavailability. Permeation enhancers generally function by opening the tight junctions formed between epithelial cells of the mucosal membrane, thereby allowing diffusion of therapeutic agent into and through the membrane.

Significant research has been conducted to enhance bioavailability across nasal membranes directed toward developing intranasal administration of insulin. See, for example, U.S. 5,112,804 and U.S. 7,112,561. The learning from these efforts can be applied in the formulation of desmopressin compositions to improved trans mucosal bioavailability. Generally, the enhancers used to promote insulin transport are more effective to improve trans mucosal desmopressin bioavailability as the target blood concentration of desmopressin is orders of magnitude smaller than effective insulin doses, and desmopressin is a much smaller polypeptide (MW 1069 vs. 5808).

The permeation enhancer used in the disclosed composition of the invention may include any entity that is compatible with peptide administration and facilitates absorption of the peptide across the nasal mucosal membrane. The claimed invention further comprises a permeation enhancer selected from the group consisting of macrocyclic esters, diesters, amides, diamides, amidines, diamidines, thioester, dithioester, thioamides, ketones or lactones. The macrocyclic moiety often contains at least twelve carbon atoms. Cyclopentadecalactone is the permeation enhancer claimed and is sold under the trade name CPE-215 by CPEX, Inc of Exeter, New Hampshire.

It is very difficult to predict which enhancer will work best for a given drug. For permeation enhancement of desmopressin, the actual effectiveness of an enhancer should be verified by routine experiments of a nature well known to the skilled artisan, e.g., using the porcine or rat model. The amount of cyclopentadecanolide included in the formulation component of the present invention will generally range between about 1 wt % to about 30 wt %. The precise nature and amount of enhancer will vary depending on, for example, the particular permeation enhancer or enhancer composition selected, and on the nature of other components in the formulation. Thus, the concentration of the cyclopentadecanolide within the medicament medium may be varied depending on the potency of the enhancer. The upper limit for enhancer concentration is set by toxic effect to or irritation limits of the mucosal membrane. The solubility of the enhancer within the medicament medium may also limit enhancer concentration.

The composition may be formulated as a simple, typically mildly acidic, aqueous solution of desmopressin, containing a water-soluble permeation enhancer molecule or multicomponent permeation enhancer composition. The composition is formulated as a two phase system with a hydrophobic and a hydrophilic phase. The composition of course may include other conventional components such as emulsifiers or surface active agents to aid in stabilization and enhancement of drop formation within the structure of the spray nozzle, preservatives so as to enhance shelf life or permit room temperature storage, stabilizers, osmolarity controls (salts), and a buffer or a buffer system. Formulations are best optimized empirically. Any given candidate formulation may be tested by intranasal administration to experimental animals, e.g., pigs or rats, or with proper approvals after appropriate pre clinical testing, to humans. Periodic sampling of blood will reveal the desmopressin concentration at various times post administration so as to permit calculation of Cₘₐₓ and other variables and the consistency of delivery to the circulation among successive doses both inter patient and intra patient.

### Example of formulation testing protocol

This example describes how to test a given candidate formulation for efficiency in transport across nasal membranes. It assumes testing of compositions comprising water soluble permeation enhancers "A" and "B" and seeks to measure the fraction of desmopressin that permeates the nasal mucosa and enters the bloodstream in a low dose range, and how this bioavailability is altered as a function of the identity and concentration of these different enhancers.

Thus, by way of example, four formulations may be prepared having the following compositions.

**Nasal formulation test compositions**

| Formulation | 1 | 2 | 3 | 4 |
|---|---|---|---|---|
| Desmopressin (µg/ml) | 2 | 2 | 2 | 2 |
| Na₂HPO₄ (mM) | 16 | 16 | 16 | 16 |
| Citric acid (mM) | 8 | 8 | 8 | 8 |
| NaCl (mM) | 145 | 145 | 145 | 145 |
| pH | 4.9 | 4.9 | 4.9 | 4.9 |
| Permeation enhancer mg/ml | "A" 2 mg/ml | "A" 10 mg/ml | "B" 2 mg/ml | "B" 10 mg/ml |

A 10µl drop of each formulation will contain 0.02 µg (20 ng) of desmopressin. A drop of a each candidate composition is applied to a nostril in each of three anesthetized rats, weighing, for example, between 225 and 250 grams. Blood is drawn prior to dosing and at 10, 20, 40, 60, and 120 minutes after dosing. The desmopressin concentration of each blood sample is determined using, for example, an immunoassay with sufficient sensitivity at the low pg desmopressin concentrations in the samples. From these data Cₘₐₓ can be calculated for each formulation and all compositions tested can be rated for efficient passage of desmopressin across rat nasal mucosal tissue. Promising formulations can be tested further, e.g., by introduction of a spray of a given formulation, volume and desmopressin concentration into the nostril of test pigs. Again, blood samples are drawn and Cₘₐₓ, AUC, or other measures of drug bioavailability can be determined. These data, in turn, permit preparation of test formulations for use in a phase I clinical trial, with the goal of designing a safety dispenser which when used correctly consistently produces a desmopressin drug concentration within a low dose target concentration range.

### Exemplary formulation

Emulsion Stock Solution To produce an emulsion stock solution, the following ingredients in parts by weight are added to a vessel equipped with a stirring bar, and mixed for 15 minutes at 60-65 °C.
180 parts sorbitan monolaurate (Span-20) aqueous solution (12 mg/ml)
30 parts Polysorbate 20 (Tween-20) aqueous solution (2 mg/ml)
400 parts cottonseed oil aqueous emulsion (26.6 mg/ml)
600 parts cyclopentadecanolide (CPE-215) aqueous emulsion (40 mg/ml)
Water to produce 1,500 grams total batch size
After mixing the preparation is homogenized using a high speed mixture at 6500 RPM+ for 20-25 minutes to produce a fine emulsion. This solution is autoclaved to assure sterility.

Buffer Solution To produce a citric acid buffer stock solution, the following ingredients in parts by weight are added to a vessel equipped with a stirring bar, and mixed for 5 minutes at 60-65 °C.
6200 parts water
16 parts anhydrous citric acid aqueous solution (1.85 mg/ml)
76 parts sodium citrate, dihydrate aqueous solution (8.9 mg/ml)
104 parts Polysorbate 20 (Tween-20) aqueous solution (12 mg/ml)
Water to produce 8,500 grams total batch size

Desmopressin Solution To produce a desmopressin stock solution, 0.111 part desmopressin acetate trihydrate is added to sufficient buffer stock solution to produce 100.0 ml of solution, and stirred until all the desmopressin is dissolved to produce a stock solution having a concentration of 100 µg desmopressin/ml. From this stock solution a 10 µg/ml solution was prepared by dilution.

Aliquots of the 10 µg/ml solution were filtered to eliminate any bacterial contamination and diluted with an equal volume of emulsion stock solution to produce aseptic, preservative free dose forms comprising 5 µg/ml desmopressin, pH 5.5, containing 2% cyclopentadecanolide. These were bottled in sterile pump spray bottles fitted with a Pfeiffer APF pump sprayers that deliver 100 µl per metered spray, or 0.50 µg desmopressin, or 500 ng desmopressin per spray. The liquid contains no detectable microorganisms. The commercially available, disposable Pfeiffer APF pump comprises a mechanism that prevents backfill of potentially contaminated air after the pump has been actuated and thus maintains substantial sterility of each dose dispensed. These were tested on humans to determine the blood concentration they delivered, duration of antidiuresis, their pharmacokinetic properties, etc., as set forth below.

### Clinical Testing of Prototype Product

A clinical study using a safety dispenser embodying the invention described above in human adult subjects in a water loaded state demonstrated that doses administered intranasally of 500 ng to 2000 ng (one to four sprays) produced antidiuretic effects in a dose proportional relationship for durations of from 2 to 7 hours. Peak blood concentrations ranged from about 1.25 to about 10 pg/ml. None of the test subjects exhibited any drug related decreases in serum sodium.

The open-label preliminary study of the effects and pharmacokinetics of the prototype dispensers was conducted with six male and six female healthy, water loaded, non-smoking volunteer subjects, following the protocol described generally below. In summary, each subject was dosed up to four times over a period of one week with dosing administered every other day. On days one, three, and five subjects were dosed intranasally with escalating doses of the low dose desmopressin nasal spray formulation described above. On day seven, subjects were given a single bolus injection of low dose desmopressin either intradermally or subcutaneously as a comparison. All subjects were screened prior to the first treatment, including evaluations of medical history, complete physical exam including nasopharyngeal exam, serum chemistry including serum osmolality, urinalysis including urine osmolality.

On day one all subjects were asked to have his/her morning void prior to breakfast, and thereafter subjects started the water loading process. Water loading assures that a patient is not generating endogenous vasopressin, and accordingly permits isolation of the effect of exogenous desmopressin. To achieve a steady state diuresis, the subjects were directed to drink a volume of water corresponding to at least 1.5% and up to 3% of body weight. The water loading process started about two hours prior to the dosing of the first subject. Subjects were asked to void every 20 minutes. To ensure continuous water loaded state, the subjects replaced their urinary output loss with an equivalent amount of fluid. Insensible loss was not measured or replaced. When the urinary output rate exceeded 10 ml/min in two consecutive measurements (defined as water loaded state) in the subjects, dosing began. Subjects were maintained in the water loaded state with equivalent fluid intake versus fluid loss.

Each subject then was dosed intranasally with one spray (100 µl containing 0.5 µg of desmopressin) of the nasal spray formulation in the right or left nostril. Urine volume was measured in 20-minute intervals from the start of water loading (at least two hours prior to dosing) to the time the subject's urine output returns to baseline (urinary output level that exceeds 10 mL/minute in three consecutive 20-minute measurements) post dose. Serum osmolality and sodium were measured prior to dosing and at 2, 4, 6 and 8 hours post dose.

Blood sampling for pharmacokinetic determinations was performed at 1, 1.5, 2, 3, 4 and 6 hours post dose. Two seven ml blood samples were collected at each time point. The concentration of desmopressin was determined by a validated radio immunoassay. The concentration of desmopressin in plasma was analyzed for the individual volunteer in each group, by use of non-compartmental methods using the commercially available software WinNonlin^{™}Pro, ver. 3.2 (Pharsight Corporation, USA). A plasma concentration value below the limit of quantitation ("LOQ") followed by values above the limit was set at 'LOQ/2' for the analysis and for the descriptive statistics on concentrations. Values below LOQ not followed by values above the LOQ were excluded from the analysis, and set to zero in the descriptive statistics on concentrations.

On days two, four, and six the subjects fasted beginning at 8 pm until breakfast the following day and were encouraged to drink one to two liters of water between 7 pm and 9 pm. Thereafter, they were to drink fluid *ad libitum* until the start of the water loading on the next day.

On day three, the subjects received one spray of desmopressin nasal spray formulation in each nostril (total volume of 200 µl equivalent to 1000 ng of desmopressin). Other than the dose level, all procedures were the same as those described for day one.

On day five, all subjects received a total volume of 2000 ng of desmopressin (one nasal spray in each nostril followed five minutes later by a second spray in each nostril). Other than the dose level, all procedures were the same as those described for day one.

On day seven, three male and three female subjects received a single bolus intradermal injection of desmopressin solution (150 µl of 0.8 µg/ml solution equivalent to 120 ng of desmopressin), and the other six subjects received a single bolus subcutaneous injection of desmopressin (150 µl of 0.8 µg/ml solution equivalent to 120 ng of desmopressin). Other than the dosing paradigm, all procedures were the same as described on day one.

Pharmacokinetic parameters were derived from the individual concentration of desmopressin found in blood samples versus time curves of desmopressin, included AUC, and Cₘₐₓ. Assay values below the limit of detection of the desmopressin immunoassay (<1.25 pg/ml) were set equal to zero for purposes of averaging concentrations. Assay values below the level of detection that occurred between two non-zero concentrations were considered to be "missing" for purposes of calculating the AUC. Blood concentration measurements from the 0.5 µg dose study were not conducted as often unreliable and below the limit of detection. Since the traditional analysis resulted in many subject/treatment combinations not being evaluable for T_{1/2} or AUC, a hypothesis was made that for a given subject, the half-life would be consistent from treatment to treatment. Therefore, as long as one of the three treatments generated an evaluable terminal half-life, that value could be used to extrapolate the AUC for the treatments that did not have evaluable half-lives. Accordingly, an average terminal half-life (Avg T_{1/2}) was calculated for each subject that included a treatment with evaluable half-lives in that subject. Ten of the twelve subjects had half-lives evaluable for at least one treatment. The AUC could be calculated for each treatment and subject using the calculated average T_{1/2} value.

It was determined that aside from one anomalous patient, all 11 patients in the study had peak desmopressin drug concentrations at the 2000 ng dose level of between 3.9 and 10 pg/ml. Furthermore, 9 of the 11 achieved drug concentrations between 5.18 and 8.4 pg/ml. This alone illustrates the consistency of the blood concentration achieved using the prototype dispenser described above. Furthermore, as a result of the study, the following AUC and Cₘₐₓ values were calculated. The calculated coefficient of variation of each data point is indicated in parentheses.

| | 1000 ng Nasally (2 sprays) | 2000 ng Nasally (4 sprays) | 120 ng Subcutaneous Injection | 120 ng Intradermal Injection |
|---|---|---|---|---|
| Cₘₐₓ pg/ml | N=7 | N=12 | N=6 | N=6 |
| | 2.79 ± 1.44 (51.6%) | 6.24 ± 2.25 (36.0%) | 2.77 ± 0.98 (35.4%) | 1.93 ± 0.46 (23.8%) |
| AUC pg·hr/ml | N=10 | N=10 | N=6 | N=4 |
| | 5.36 ± 5.92 (110.5%) | 11.59 ± 7.9 (68.0%) | 7.85 ± 4.21 (53.6%) | 4.46 ± 3.09 (69.4%) |
| T_{1/2} hr | N=3 | N=8 | N=3 | N=2 |
| | 1.13 ± 0.30 (26.3%) | 1.33 ± 0.56 (42.3%) | 2.09 ± 0.32 (15.4%) | 1.39 ± 0.61 (43.5%) |

Two conclusions may be derived from these data. First, the coefficient of variation of Cₘₐₓ of desmopressin administered intranasally using the safety dispenser of the invention for the 1000 ng dose (51.6%) is only about 30% greater than coefficient of variation of Cₘₐₓ of a dose of desmopressin administered *subcutaneously* and designed to produce comparable low blood concentrations. The measured coefficient of variation of Cₘₐₓ of desmopressin administered intranasally using the dispensed composition of the invention for the 2000 ng dose (36.0%) is about equal to the coefficient of variation of Cₘₐₓ of the subcutaneous dose. These preliminary data support the hypothesis that the formulation of the invention indeed is characterized by a coefficient of variation of Cₘₐₓ comparable to that of subcutaneous desmopressin doses designed to achieve a comparable low blood concentration. This is in sharp contrast to commercially available intranasal desmopressin dose forms which, despite being designed to deliver far higher blood concentrations, have a much higher variation in Cₘₐₓ, a variation that contributes to the stochastic induction of a hyponatremic state.

Second, note that both AUC and Cₘₐₓ produced by this formulation dispensed intranasally in accordance with the invention appear to be directly linearly proportional to dose. Thus, the 1000 ng intranasal dose yields a Cₘₐₓ of 2.79 +/- 1.44 pg/ml, while the 2000 ng dose yields a value of 6.24 +/- 2.25; the 1000 ng intranasal dose results in an AUC of 5.36+/- 5.92,which is approximately doubled to 11.59+/-7.9 when the dose is doubled. This suggests that desmopressin can be reliably dispensed intranasally to reproducibly achieve an antidiuretic effect of limited duration without substantial risk of members of a patient population developing hyponatremia. It also suggests that a dispenser delivering a low dose may be used via multiple sprays to achieve any of several antidiuresis durations in a given patient, or that one dispenser may be sold to service different patient populations provided there is proper instruction for how many sprays should be used to produce a given duration of effect in a given population.

The results of this study suggest that the low-dose desmopressin nasal spray embodying the invention provides improved, more reproducible pharmacokinetic parameters at relatively consistent low blood concentrations, and delivers a Cₘₐₓ proportional to the doses administered.

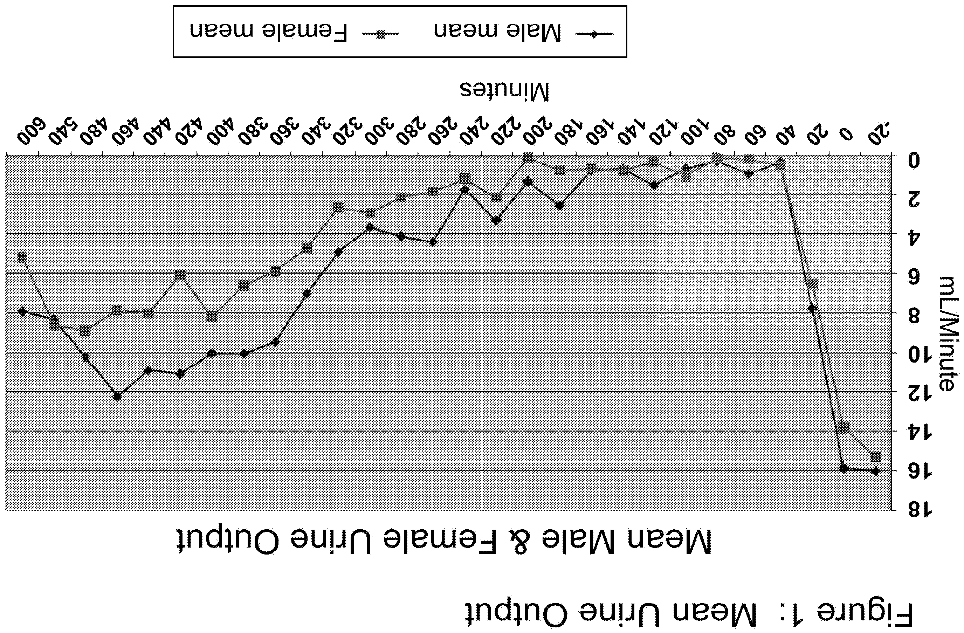

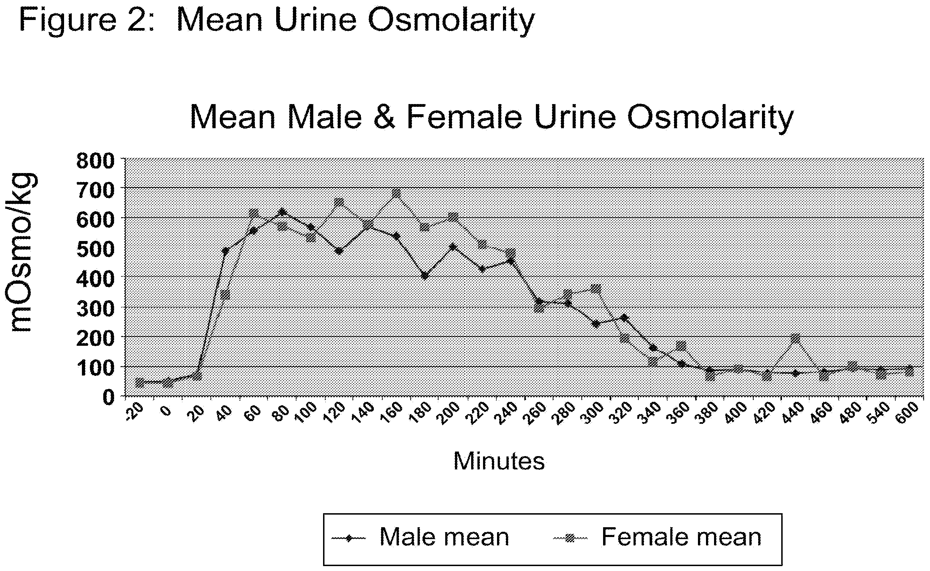

The urine output and urine osmolarity was measured just prior to nasal administration of 2000 ng of the pharmaceutical composition of desmopressin and for a period of up to about 10 hours (600 minutes) after administration. Figure 1 shows the mean urine output for male and female subjects. As evidenced by the data, the urine output fell to less than 8 ml/minute within 20 minutes after administration of the desmopressin by nose (in water loaded individuals). Urine output remained less than 8 ml/minute for a period ranging up to about 400 minutes after administration. Figure 2 shows the mean urine osmolarity for the same group of male and female subjects as in Figure 1. Urine osmolarity increased to greater than about 400 mOsmol/kg within 40 minutes after administration of 2 µg of desmopressin nasally and remained greater than about 400 mOsmol/Kg for about 250 minutes after administration of the desmopressin by nose.

A second separate study in adult patients with nocturia established that doses of 500 and 1000 ng (one or two sprays administered intranasally) produced dramatic therapeutic decreases in the number of night time urinary voids equal to or less than one per night in 41 of 43 patients. Serum sodium levels remained within normal limits throughout treatment.

Other embodiments are within the following claims.

## Claims

1. A safety dispenser for inducing in members of a patient population an antidiuretic effect while reducing the risk that a member of the population may develop hyponatraemia, the dispenser comprising: a reservoir having disposed therein a composition formulated as a two phase system with a hydrophobic and a hydrophilic phase comprising a desmopressin preparation and a nasal membrane permeation enhancer comprising cyclopentadecanolide aqueous emulsion in an amount sufficient to constitute multiple drug doses, said composition **characterized by** a desmopressin bioavailability of 5% to 25%; an outlet in communication with said reservoir; a manually actuatable pump for serially dispensing from said reservoir through said outlet, and to an intranasal surface of a patient, measured doses of said composition in the form of a spray, respective doses of spray being restricted within the range of 0.5ng desmopressin per kilogram of the patient's body weight and 75 ng desmopressin per kilogram of the patient's body weight so as to achieve a target Cₘₐₓ in members of said patient population less than 15 +/- 3 pg/ml of blood, said dispenser being sufficient to establish in a said patient by drug transport across intranasal mucosal membranes delivery of blood concentrations of desmopressin in the range below 15 +/- 3 pg/ml substantially directly proportional to the mass of desmopressin dispensed into the nostril(s) of a said patient.

2. The safety dispenser of claim 1 wherein the dispenser dispenses a metered spray dose comprising a multiplicity of droplets of said composition with an average volume distribution in the range of 20 µm for D10 to 300 µm for D90.

3. The dispenser of any one of the preceding claims further comprising means for blocking dispensing of a second said desmopressin dose from said reservoir for a time interval after dispensing a said dose.

4. The dispenser of any one of the preceding claims formulated: (a) to induce antidiuresis in a target patient population for less than six hours; (b) to induce antidiuresis in a target patient population for between 2 and 4 hours; (c) to induce antidiuresis in a target patient population for between 4 and 7 hours; (d) for a target population selected from children, children weighing less than 35 kg, children weighing between 35 and 50 kg, adult females, adult males, females weighing between 50 and 75 kg, males weighing between 70 and 85 kg, and males weighing more than 85 kg.

5. The dispenser of any one of the preceding claims wherein said composition is **characterized by** a desmopressin bioavailability greater than 10%, greater than 15% or greater than 20%.

6. The dispenser of any one of the preceding claims being free of preservatives.

7. The dispenser of any one of the preceding claims wherein said pump prevents backfill of bacterially contaminated ambient air after a dispensing of said composition.

8. The dispenser of any one of the preceding claims wherein said dispenser establishes in a said patient by drug transport across intranasal mucosal membranes delivery of blood concentrations of desmopressin directly proportional to the mass of desmopressin dispensed into the nostril(s) of a said patient.

9. A metered spray dose dispensed by a dispenser of any one of the preceding claims for use in a method of inducing an antidiuretic effect in a patient, the method comprising intranasally administering to the patient desmopressin from said dispenser.

10. The dispensed dose for use according to claim 9 wherein said method: (a) produces a desmopressin concentration no greater than 15 +/- 3 pg/ml in the bloodstream of the patient; (b) produces a desmopressin concentration no greater than 10 +/- 3 pg/ml in the bloodstream of the patient; (c) produces a desmopressin concentration no greater than 7 +/- 3 pg/ml in the bloodstream of the patient; (d) induces antidiuresis for less than six hours; (e) induces antidiuresis for between 2 and 4 hours; or (f) induces antidiuresis for between 4 and 7 hours.

## Patentansprüche

1. Sicherheitsspender, der bei Mitgliedern einer Patientenpopulation eine antidiuretische Wirkung induziert und gleichzeitig das Risiko verringert, dass ein Mitglied der Population eine Hyponatriämie entwickelt, wobei der Spender umfasst: ein Reservoir, in dem eine Zusammensetzung angeordnet ist, die als Zwei-Phasen-System mit einer hydrophoben und einer hydrophilen Phase formuliert ist, die eine Desmopressinpreparation und einen nasalen Membranpermeationsverstärker umfasst, der eine wässrige Cyclopentadecanolidemulsion in einer Menge umfasst, die ausreicht, um mehrere Wirkstoffdosen zu bilden, wobei die Zusammensetzung durch eine Desmopressinbioverfügbarkeit von 5 % bis 25 % gekennzeichnet ist; einen Auslass in Verbindung mit dem Reservoir; eine manuell betätigbare Pumpe zur seriellen Ausgabe aus dem Reservoir durch den Auslass und auf eine intranasale Oberfläche eines Patienten, wobei die gemessenen Dosen der Zusammensetzung in Form eines Sprays vorliegen, wobei die jeweiligen Dosen des Sprays auf einen Bereich zwischen 0,5 ng Desmopressin pro Kilogramm des Körpergewichts des Patienten und 75 ng Desmopressin pro Kilogramm des Körpergewichts des Patienten begrenzt sind, um ein Ziel Cₘₐₓ in Mitgliedern der Patientenpopulation zu erreichen, das geringer als 15 +/- 3 pg/ml Blut ist, wobei der Spender ausreicht, um in einem Patienten durch Arzneimitteltransport über intranasale Schleimhautmembranen die Lieferung einer Blutkonzentrationen von Desmopressin im Bereich unter 15 +/- 3 pg/ml im Wesentlichen direkt proportional zu der Masse von Desmopressin, das in das oder die Nasenlöcher eines Patienten abgegeben wird, herbeizuführen.

2. Sicherheitsspender nach Anspruch 1, wobei der Spender eine dosierte Sprühdosis abgibt, die eine Vielzahl von Tröpfchen der Zusammensetzung mit einer durchschnittlichen Volumenverteilung im Bereich von 20 µm für D10 bis 300 µm für D90 umfasst.

3. Spender nach einem der vorhergehenden Ansprüche, der ferner Mittel zum Sperren der Abgabe einer zweiten Desmopressindosis aus dem Reservoir für einen Zeitraum nach dem Verabreichen einer Dosis umfasst.

4. Spender nach einem der vorhergehenden Ansprüche, ausformuliert: (a) zum Induzieren einer Antidiurese in einer Zielpatientenpopulation für weniger als 6 Stunden; (b) zum Induzieren einer Antidiurese in einer Zielpatientenpopulation für 2 bis 4 Stunden; (c) zum Induzieren einer Antidiurese in einer Zielpatientenpopulation für 4 bis 7 Stunden; (d) für eine Zielpopulation, die ausgewählt ist aus Kindern, Kindern, die weniger als 35 kg wiegen, Kindern, die zwischen 35 und 50 kg wiegen, erwachsenen Frauen, erwachsenen Männern, Frauen, die zwischen 50 und 75 kg wiegen, Männern, die zwischen 70 und 85 kg wiegen, und Männern, die mehr als 85 kg wiegen.

5. Spender nach einem der vorhergehenden Ansprüche, wobei die Zusammensetzung durch eine Desmopressinbioverfügbarkeit größer als 10%, größer als 15 % oder größer als 20 % gekennzeichnet ist.

6. Spender nach einem der vorhergehenden Ansprüche, der frei von Konservierungsstoffen ist.

7. Spender nach einem der vorhergehenden Ansprüche, wobei die Pumpe ein Rückfüllen von bakteriell verunreinigter Umgebungsluft nach einer Abgabe der Zusammensetzung verhindert.

8. Spender nach einem der vorhergehenden Ansprüche, wobei der Spender in einem Patienten durch Arzneimitteltransport über intranasale Schleimhautmembranen die Lieferung von Blutkonzentrationen von Desmopressin direkt proportional zu der in das Nasenloch oder die Nasenlöcher eines Patienten abgegebenen Masse von Desmopressin bewirkt.

9. Dosierte Sprühdosis, die durch einen Spender nach einem der vorhergehenden Ansprüche zur Verwendung in einem Verfahren zum Induzieren eines antidiuretischen Effekts bei einem Patienten abgegeben wird, wobei das Verfahren die intranasale Verabreichung von Desmopressin aus dem Spender an den Patienten umfasst.

10. Abgabedosis zur Verwendung nach Anspruch 9, wobei das Verfahren: (a) eine Desmopressinkonzentration erzeugt, die nicht größer als 15 +/- 3 pg/ml in dem Blutstrom eines Patienten ist; (b) eine Desmopressinkonzentration erzeugt, die nicht größer als 10 +/- 3 pg/ml in dem Blutstrom eines Patienten ist; (c) eine Desmopressinkonzentration erzeugt, die nicht größer als 7 +/- 3 pg/ml in dem Blutstrom eines Patienten ist; (d) eine Antidiurese für weniger als 6 Stunden induziert; (e) eine Antidiurese für 2 bis 4 Stunden induziert; oder (f) eine Antidiurese für 4 bis 7 Stunden induziert.

## Revendications

1. Distributeur sécurisé destiné à induire chez des membres d'une population de patients un effet antidiurétique tout en réduisant le risque de développer une hyponatrémie chez un membre de la population, le distributeur comprenant : un réservoir dans lequel est disposée une composition formulée comme un système à deux phases ayant une phase hydrophobe et une phase hydrophile comprenant une préparation de desmopressine et un agent améliorant la perméation de membrane nasale comprenant une émulsion aqueuse de cyclopentadécanolide en une quantité suffisante pour constituer des doses multiples de médicament, ladite composition étant **caractérisée par** une iodisponibilité de desmopressine allant de 5 % à 25 % ; une sortie en communication avec ledit réservoir ; une pompepouvant être actionnée manuellement pour distribuer en série à partir dudit réservoir à travers ladite sortie, et à une surface intranasale d'un patient, des doses mesurées de ladite composition sous la forme d'une pulvérisation, des doses respectives de pulvérisation étant limitées dans la plage comprise entre 0,5 ng de desmopressine par kilogramme du poids corporel du patient et 75 ng de desmopressine par kilogramme du poids corporel du patient de manière à atteindre une Cₘₐₓ cible chez les membres de ladite population de patients inférieure à 15 +/- 3 pg/ml de sang, ledit distributeur étant suffisant pour établir chez ledit patient par transport de médicament à travers les membranes muqueuses intranasales, l'administration de concentrations sanguines de desmopressine dans la plage inférieure à 15 +/- 3 pg/ml essentiellement directement proportionnelles à la masse de desmopressine distribuée dans la/les narine(s) dudit patient.

2. Distributeur sécurisé de la revendication 1, le distributeur distribuant une dose de pulvérisation mesurée comprenant une multiplicité de gouttelettes de ladite composition avec une distribution de volume moyenne dans la plage allant de 20 µm pour D10 à 300 µm pour D90.

3. Distributeur de l'une quelconque des revendications précédentes, comprenant en outre des moyens pour bloquer la distribution de ladite deuxième dose de desmopressine à partir dudit réservoir pendant un intervalle de temps après la distribution de ladite dose.

4. Distributeur de l'une quelconque des revendications précédentes formulé : (a) pour induire une antidiurèse chez une population de patients cible pendant moins de six heures ; (b) pour induire une antidiurèse chez une population de patients cible pendant une durée allant de 2 à 4 heures ; (c) pour induire une antidiurèse chez une population de patients cible pendant une durée allant de 4 à 7 heures ; d) pour une population cible choisie entre des enfants, des enfants pesant moins de 35 kg, des enfants pesant entre 35 et 50 kg, des femmes adultes, des hommes adultes, des femmes pesant entre 50 et 75 kg, des hommes pesant entre 70 et 85 kg, et des hommes pesant plus de 85 kg.

5. Distributeur de l'une quelconque des revendications précédentes, dans lequel ladite composition est **caractérisée par** une biodisponibilité de desmopressine supérieure à 10 %, supérieure à 15 % ou supérieure à 20 %.

6. Distributeur de l'une quelconque des revendications précédentes, qui est exempt d'agents de conservation.

7. Distributeur de l'une quelconque des revendications précédentes, dans lequel ladite pompe empêche le remplissage de l'air ambiant contaminé par des bactéries après une distribution de ladite composition.

8. Distributeur de l'une quelconque des revendications précédentes, ledit distributeur établissant chez ledit patient, par transport de médicament à travers les membranes muqueuses intranasales, l'administration de concentrations sanguines de desmopressine directement proportionnelles à la masse de desmopressine distribuée dans la/les narine(s) dudit patient.

9. Dose de pulvérisation mesurée distribuée par un distributeur de l'une quelconque des revendications précédentes, destinée à être utilisée dans un procédé pour induire un effet antidiurétique chez un patient, le procédé comprenant le fait d'administrer par voie intranasale, au patient, la desmopressine à partir dudit distributeur.

10. Dose distribuée destinée à être utilisée selon la revendication 9, ledit procédé : (a) produisant une concentration de desmopressine non supérieure à 15 +/- 3 pg/ml dans la circulation sanguine du patient ; (b) produisant une concentration de desmopressine non supérieure à 10 +/- 3 pg/ml dans la circulation sanguine du patient ; (c) produisant une concentration de desmopressine non supérieure à 7 +/- 3 pg/ml dans la circulation sanguine du patient ; (d) induisant une antidiurèse pendant moins de six heures ; (e) induisant une antidiurèse pendant une durée allant de 2 à 4 heures ; ou (f) induisant une antidiurèse pendant une durée allant de 4 à 7 heures.
